Europäisches Patentamt

European Patent Office

Office européen des brevets

(11) Publication number: **0 243 250**
**B1**

(12) **EUROPEAN PATENT SPECIFICATION**

(45) Date of publication of patent specification: **06.02.91**

(21) Application number: **87400872.5**

(22) Date of filing: **16.04.87**

(51) Int. Cl.⁵: **A 61 F 13/20**

(54) **Curved grippable tampon applicator.**

(30) Priority: **23.04.86 US 855048**

(43) Date of publication of application:
**28.10.87 Bulletin 87/44**

(45) Publication of the grant of the patent:
**06.02.91 Bulletin 91/06**

(84) Designated Contracting States:
**DE FR GB**

(56) References cited:
**DE-A-2 260 940**
**US-A-2 704 068**
**US-A-3 765 417**
**US-A-4 536 178**

(73) Proprietor: **KIMBERLY-CLARK CORPORATION**
**401 North Lake Street**
**Neenah Wisconsin 54956 (US)**

(72) Inventor: **Paul, Susan C.**
**1337 Meadow Lane**
**Neenah Wisconsin (US)**
Inventor: **Sheldon, Donald A.**
**926 West Roberts Avenue**
**Appleton Wisconsin (US)**

(74) Representative: **Sauvage, Renée**
**Cabinet Sauvage 100 bis, avenue de Saint-Mandé**
**F-75012 Paris (FR)**

Courier Press, Leamington Spa, England.

## Description

### Field of the invention

This invention relates to an improvement in tampon applicators. It particularly relates to a pair of telescoping elements having a tampon disposed in the forward portion of the outer element. The outer element is provided with a gripping means in an oval cross section to aid the user in holding of the device during insertion of the tampon. Also the forward end of the outer element has petals to aid in the insertion of the tampon.

### Prior art

There are a variety of catamenial tampon applicators on the market. Many of these devices comprise telescoping elements in which the outer element is a tubular member adapted to contain the tampon and the inner element comprises a pusher member for expelling the tampon. The pusher member may be in the form of a tube or may be solid, such as a stick. The devices on the market are generally designed such that the axis of both the tube and pusher is straight when the members are in an operating position.

It has been proposed in US—A—4 536 178 an applicator for a preformed tampon disposed therein; said applicator comprising a pair of straight tubular members in telescoping association including an outer member adapted to contain a tampon in the leading end thereof and an inner member disposed in the trailing end of said outer member and adapted to eject said tampon from said outer member by a telescopic movement therein, wherein said outer tube is provided with a gripping means at its trailing end, wherein said gripping means has a reduced cross-sectional area from said leading end and wherein said gripping means has an oval cross section.

It has also been proposed in United States Patent 3,765,417—Crockford—that a tampon applicator be formed having telescoping elements in an arcuate shape. Further, it is disclosed therein that the elements may have an oval cross section in both the inner and outer tube. The arc of the radius of the tampon of Crockford is disclosed as between about 101.6 to about 152.4 mm (4 to about 6 inches). This tampon applicator has a disadvantage that the extreme curvature may make the product hazardous, inconvenient or difficult to handle and to insert with such a sharp a curve. A further disadvantage is that the increased possibility that a sharply curved tampon having petals on the end would have the petals close upon the flesh of the vagina after insertion of the tampon. This is a greater problem for a curved petal end product in that it may actually scratch the vaginal walls, not just the tissue of the labia minora.

There remains a need for an improved tampon that is convenient and comfortable to insert, provides good gripping during insertion and is inexpensive but reliable.

### Invention

In the tampon application system of the invention there is provided an arcuate tampon applicator having an inner end outer tube. Both the inner tube and the outer tube finger grip section have an oval cross section. The outer tube is provided with a gripping section at the outer end that both provides a place for the user to grip and also provides a narrow cross section to prevent the withdrawal of the inner tube that has an expanded forward end portion. The tampon assembly of the invention further is provided with petals on the forward end that aid in providing easy insertion of the device into the vagina. The applicator has a curve corresponding to the arc of a circle having a radius of between about 203.2 and about 254 mm (about 8 and about 10 inches). Further, the oval is that formed by an elipse of between 15° and about 45°. The barrel end on the section that holds the tampon may be round or oval in cross section and if oval, the oval is that formed by an elipse of between 15° and about 45°.

### Brief description of the drawings

Figure 1 is a sectional view of an outer tube in accordance with the invention.

Figure 2 is the end view of the outer tube of the invention.

Figure 3 is a cross-sectional view of the outer tube of Figure 1 on section line 3—3.

Figure 4 is a cross sectional view on line 4—4 of Figure 2.

Figure 5 is a side sectional view of the inner tube of the applicator assembly of the invention.

Figure 6 is an end view of the inner tube of Figure 5.

Figure 7 is a sectional view of the applicator of the invention.

Figure 8 is a sectional view of the applicator of the invention in the position where the tampon is ejected from the applicator.

### Detailed description of the invention

The tampon applicator of the invention has numerous advantages over the prior practices and applicators. The applicator of the invention is compact and easy to use. Further, the curve is such that it is perceived as comfortable during insertion and ejection of the tampon. The oval cross section of the finger grip section makes it easy to hold with the curve in the best position for insertion. The radius of curvature is such that it is comfortable to use and not perceived as hazardous. The combination of the movable inner tube and gripping portion allows easy one-hand insertion by a technique such as that used to eject the material from a syringe. These and other advantages of the invention will be apparent from the detailed description of the invention below.

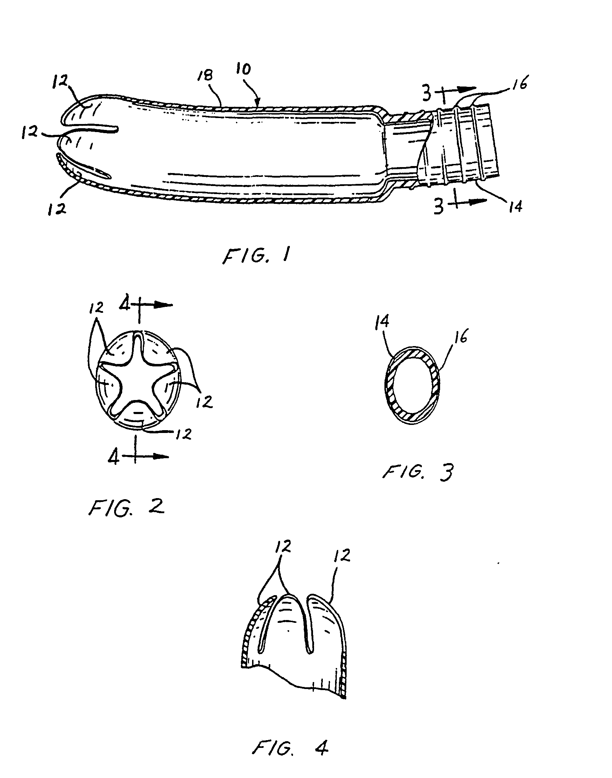

Illustrated in Figure 1, in cross sectional view, is the outer tampon tube of the tampon applicator of the invention. The tube 10 is provided with petals 12. While illustrated with five petals, it is also possible that a differing number of petals could be utilized. Five petals are preferred because an odd number of petals would prevent collapse at 180° or a flattening of the applicator which could cause additional pinching or scratching. The outer tube is provided with gripping portion 14. Gripping portion 14 is provided with ridges 16 that aid in gripping the tube between the fingers during insertion. While shown with ridges, the tube also could be provided with other gripping means such as raised protuberances, bumps or a gnarled pattern. The mid portion of the wall of the outer tube 18 is formed of as thin a material as possible. Preferred thickness is about 0.381 mm (.015 inches) for low cost formation with minimum use of plastic. However, any desired wall thickness may be used. The thickness of the gripping portion 14 is somewhat thicker being preferably about 0.889 mm (0.035 inches) for strength to support gripping while the inner tube is sliding within the gripping portion. The overall dimensions of the outer tube are between about 63.5 and about 76.2 mm (2-1/2 and about 3 inches) long. Preferably the length is about 69.85 mm (2-3/4 inches) for ease of insertion and discrete storage. The width of the wider portion 18 may be between about 12.7 mm and about 19.05 mm (1/2 and about 3/4 inches). The preferred height diameter is about 15.24 mm (0.6 inches) to provide a tube of adequate volume while not being so large as to make insertion of the tampon applicator uncomfortable.

As illustrated in Figure 2, the petals 12 do not meet in the center and have spaces between them. This design allows for easier ejection of the tampon as well as not pinching of the vagina when the petals come towards each other again after tampon ejection from the tube. Generally there is about 6.35 mm (1/4 inch) diameter circular opening between the petals, although a lesser degree of openness may be employed depending upon the tube-forming process. Figure 3 illustrates the oval cross section of the gripping portion 14 of the outer tube. The oval generally is an elipse of between about 15° and about 45°. Preferably the elipse is about 30° for good gripping and also providing a cross section resulting in a storng tube. By a 30° elipse it is meant that its cross-section is the way a cricle would be viewed when turned 30° from the viewing eye. The wider portion of the tube 18 may also be oval in cross-sectional configuration although this is not necessary. A circular cross section would also be suitable for the wider portion of the outer tube. It is particularly desirable that the gripping portion be eliptical in order to provide a tactile method of the user maintaining the curve in the proper orientation for insertion. This also prevents the inner tube from rotating 180° out of phase with the outer tube which would make expulsion of the pledget very difficult. Other methods of preventing this from happening could include round tubular sections with slots or other mechanisms similar to a key in a lock.

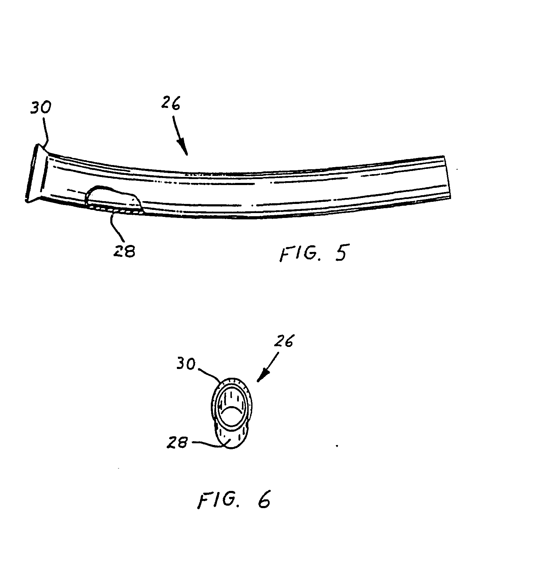

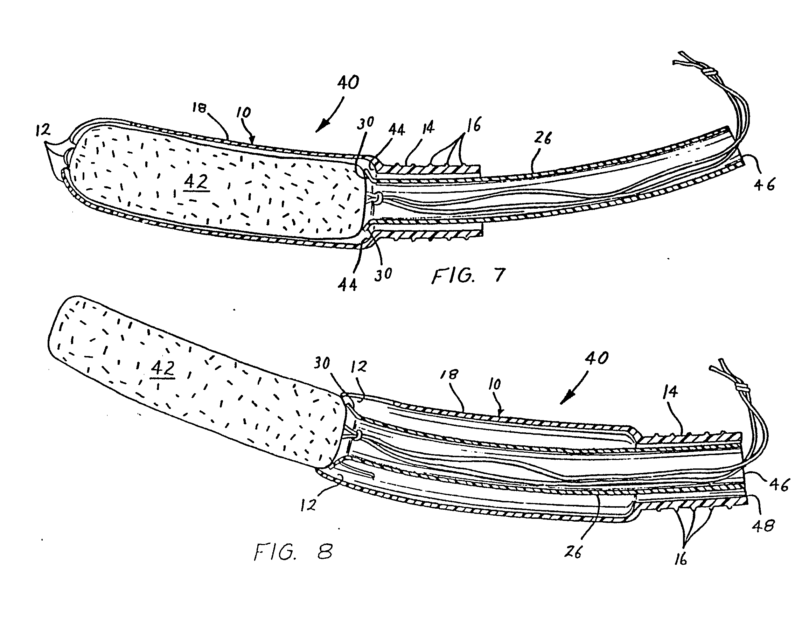

Figure 5 is a side cross-sectional view of the inner tube 26 of a tampon applicator in accordance with the invention. The tube 26 has a gently curved shape and an oval cross section as illustrated in Figure 6. The tube wall thickness is as thin as possible for a tube that will be strong enough to allow insertion of the tampon. Generally a wall thickness of about 0.254 mm (0.01 inch) is sufficient for a tube of high density polyethylene or polypropylene. The tube has a smooth oval cross section through the body portion 28 and a flared end 30. The flared end 30 serves to prevent withdrawal of the inner tube from the applicator. Correspondingly the bottom end may be flared to prevent the inner tube from being separated from the outer tube during tampon expulsion. Figure 7 is a cross-sectional illustration of the tampon applicator of the invention 40. The assembly is comprised of outer tube 10 and inner tube 26. The tube has a radius of curvature on its centerline of about 203.2 mm (8 inches) and contains tampon 42. The inner tube 26 is prevented from withdrawal through the gripping portion 16 by the flared end 30 that rests against shoulder 44 of outer tube 10. Figure 8 illustrates the applicator assembly 40 in the position where the tampon has been ejected. The ejection is accomplished by grasping the gripping portion 14 between two fingers and utilizing the thumb at end 46 of inner tube 26 to displace the inner tube such that end 46 is flush with the outer end 48 of gripping portion 14. The motion is similar to that utilized in ejecting material from a syringe. The oval cross section allows the user to orientate the applicator by feel for ease of insertion and holding during use. The inner tube corresponds in length to the length of outer tube 10 such that the tampon 42 is substantially completely ejected from the outer tube 10 thorugh the open petals 12. After ejection of the tampon 42 the applicator 40 is withdrawn from the vagina and disposed of.

The material of the assembly tubes may be any desirable material that is strong enough to support the gripping and ejection activities. The preferred material is a plastic that may be formed in a thin wall flexible form. Typical of such plastics are polyethylene, polystyrene, polypropylene and acrylonitrile-butadiene-styrene resins. A preferred material is low density or linear low density polyethylene as this may be formed into thin wall tubes, is low in cost and easily molded. It is possible that different materials may be used for the inner and outer tubes. Another suitable material in the event that a flushable applicator was desired would be a cardboard convolutely wound tube held together with a water-soluble adhesive. Such an applicator tube is shown in U.S. Patent 4,522,967—Sheldon et al.

The tampon utilized for ejection from the tampon assembly of this invention may be any suitable construction. Generally, the tampons held within the preferred applicator have an absorbent property of between about 4 and 16 cm$^3$ as measured with syndate in a syngyna. The tampons preferably have a cross section corresponding to the cross section of the outer tube. If the leading end of the tube is oval in cross section, as preferred, then it is preferred that the tampon also be oval.

The curve of the applicator is the arcuate portion of a circle formed with about an 203.2 to about 254 mm (8 to about a 10 inch) radius, as this is most comfortable to use. Less of a curve is not noticeable or helpful and more of a curve is viewed by the user as being possibly harzardous and uncomfortable to use. This amount of curve according to the invention also allows tampons to be formed straight and not curved which lowers costs.

**Claims**

1. Arcuate applicator (40) for a preformed tampon (42) disposed therein; said applicator comprising a pair of tubular members in telescoping association including an outer member (10) adapted to contain a tampon (42) in the leading end thereof and an inner member (26) disposed in the trailing end of said outer member and adapted to eject said tampon from said outer member by a telscoping movement therein, said outer tube being provided with a gripping means (14) at its trailing end, said gripping means having a reduced cross-sectional area from said leading end and having an oval cross section, characterized in that the longitudinal axis of each of said outer and inner members describes an arc of the same radius, in the range of about 203.2 to about 254 mm (8 to about 10 inches), and in that said tampon (42) is straight prior to insertion into said outer tube (10).

2. The applicator of claim 1 wherein said inner tub (26) is flared (30) at its leading end.

3. The applicator of claim 1 wherein said outer tube (10) is provided with petals (12) at said leading end.

4. The applicator of claim 1 wherein said inner and said outer tubes (26, 10) are substantially the same length.

5. The applicator of claim 1 wherein said gripping means (14) is provided with surface protrusions (16) to provide a means to aid gripping.

6. The applicator of claim 1 wherein the inner tube (26) is formed of high density polyethylene and the outer tube (10) is formed of linear low density polyethylene.

7. The applicator of claim 1 wherein the oval of said gripping means (14) is a 15° to 40° elipse.

8. The applicator of claim 3 wherein there are an odd number of petals (12).

9. The applicator of claim 1 wherein said gripping means (14) forms an oval that is an elipse of between about 15° and about 45°.

10. The applicator of claim 1 wherein said leading end has an oval cross section.

11. The applicator of claim 1 wherein said leading end has a round cross section.

**Patentansprüche**

1. Cobogene Einführhilfe (40) für einen darin angeordneten, vorgeformten Tampon (42); wobei die Einführhilfe ein Paar röhrenförmiger, in teleskopischer Verbindung stehender Teile aufweist, die ein äußeres Teil (10), das zur Aufnahme eines Tampons (42) in seinem vorlaufenden Ende ausgebildet ist, und ein inneres Teil (26) enthalten, das im nachlaufenden Ende des äußeren Teiles angeordnet ist, und dazu dient, den Tampon aus dem äußeren Teil durch eine teleskopische Bewegung darin auszustoßen, wobei die äußere Röhre an ihrem nachlaufenden Ende mit einer Griffeinrichtung (14) versehen ist, wobei die Griffeinrichtung eine vom vorlaufenden Ende reduzierte Querschnittsfläche und einen ovalen Querschnitt aufweist, dadurch gekennzeichnet, daß die Längsachse sowohl des äußeren und inneren Teils einen Bogen mit dem gleichen Radius im Bereich von etwa 203,2 bis etwa 254 mm (8 bis etwa 10 inches) beschreibt, und daß der Tampon (42) vor dem Einsetzen in die äußere Röhre (10) gerade ist.

2. Einführhilfe nach Anspruch 1, wobei die innere Röhre (26) an ihrem vorlaufenden Ende trichterförmig erweitert (30) ist.

3. Einführhilfe nach Anspruch 1, wobei die äußere Röhre (10) am vorlaufenden Ende mit blumenblattförmigen Gebilden (12) versehen ist.

4. Einführhilfe nach Anspruch 1, wobei die inneren und äußeren Röhren (26, 10) im wesentlichen die gleiche Länge haben.

5. Einführhilfe nach Anspruch 1, wobei die Griffeinrichtung (14) mit Oberflächenerhebungen (16) versehen ist, um das Ergreifen zu unterstützen.

6. Einführhilfe nach Anspruch 1, wobei die innere Röhre (26) aus einem Polyäthylen hoher Dichte und die äußere Röhre (10) aus einem linearen Polyäthylen niedriger Dichte geformt sind.

7. Einführhilfe nach Anspruch 1, wobei das Oval der Griffeinrichtung (14) eine 15° bis 40° Ellipse ist.

8. Einführhilfe nach Anspruch 3, wobei eine ungerade Anzahl blumenblattartiger Gebilde (12) vorgesehen ist.

9. Einführhilfe nach Anspruch 1, wobei die Griffeinrichtung (14) ein Oval formt, das eine Ellipse von zwischen etwa 15° und etwa 45° ist.

10. Einführhilfe nach Anspruch 1, wobei das vorlaufende Ende einen ovalen Querschnitt hat.

11. Einführhilfe nach Anspruch 1, wobei das vorlaufende Ende einen runden Querschnitt hat.

**Revendications**

1. Applicateur arqué (40) pour tampon préformé (42) disposé à l'intérieur de celui-ci, ledit applicateur comprenant une paire d'éléments tubulaires en association télescopique comprenant un élément extérieur (10) adapté à contenir un tampon (42) dans l'extrémité d'attaque de celui-ci et un élément intérieur (26) disposé dans l'extrémité et fuite dudit élément extérieur et adapté à éjecter ledit tampon dudit élément extérieur par un mouvement télescopique dans celui-ci, ledit tube extérieur étant pourvu de moyens de préhension (14) à son extrémité de fuite, lesdits moyens de préhension ayant une zone de section transversale réduite depuis ladite extrémité d'attaque et ayant une section transversale ovale, caractérisé en ce que l'axe longitudinal de chacun desdits éléments extérieur et intérieur décrit un arc de même rayon, dans la gamme allant d'environ 203,2 à environ 254 mm et en ce que ledit tampon (42) est rectiligne avant son insertion dans ledit tube extérieure (10).

2. Applicateur selon la revendication 1, dans lequel ledit tube intérieur (26) est évasé (30) à son extrémité d'attaque.

3. Applicateur selon la revendication 1, dans lequel ledit tube extérieur (10) est pourvu de pétales (12) à son extrémité d'attaque.

4. Applicateur selon la revendication 1, dans lequel lesdits tubes intérieur et extérieur (26, 10) sont sensiblement de la même longueur.

5. Applicateur selon la revendication 1, dans lequel lesdits moyens de préhension (14) sont munis de protubérances superficielles (16) pour offrir un moyen facilitant la préhension.

6. Applicateur selon la revendication 1, dans lequel le tube intérieur (26) est formé de polyéthylène haute densité et en ce que le tube extérieur (10) est formé de polyéthylène basse densité linéaire.

7. Applicateur selon la revendication 1, dans lequel l'ovale desdits moyens de préhension (14) a une ellipse de 15° à 40°.

8. Applicateur selon la revendication 3, dans lequel il y a un nombre impair de pétales (12).

9. Applicateur selon la revendication 1, dans lequel lesdits moyens de préhension (14) forment un ovale qui est une ellipse d'environ 15° à environ 45°.

10. Applicateur selon la revendication 1, dans lequel ladite extrémité d'attaque a une section transversale ovale.

11. Applicateur selon la revendication 1, dans lequel ladite extrémité d'attaque a une section transversale ronde.

FIG. 1

FIG. 2

FIG. 3

FIG. 4

*30* *26*

FIG. 5

*28*

*26*

*30*

*28*

FIG. 6

FIG. 7

FIG. 8